# EUROPEAN PATENT APPLICATION

(11) **EP 1 027 864 A1**
(43) Date of publication of application: **16.08.2000**
(21) Application number: 00830089.9
(22) Date of filing: 08.02.2000
(51) Int. Cl.: A61B 5/15

(54) **Blood withdrawal device using an evacuated test-tube**

(30) Priority: 12.02.1999 IT MS990002
(71) Applicant: Cagetti, Dino, 54038 Montignoso (Massa Carrara) (IT)
(72) Inventor: Cagetti, Dino, 54038 Montignoso (Massa Carrara) (IT)
(74) Representative: Bardini, Marco Luigi

(57) **Abstract**

A device for withdrawing blood by vacutainers (14), engageable with a substantially bell-shaped holder (15), comprises a withdrawal needle (2) and a second needle (11), for piercing a vacutainer (14). The device comprises a box-shaped body (16), substantially coaxial to the bell (15), supporting at one end the withdrawal needle (2); a sealed chamber (7) defined within the body (16), comprising a wider end section (7a), communicating with the lumen of the withdrawal needle (2) and having a axial hole (12) at the opposite end thereof; and a box-shaped plunger (8) sealingly sliding within said chamber (7), supporting the second needle (11) and communicating with the related lumen, the second needle (11) extending externally to the body (16) through the axial hole (12). At a first stroke end position of the plunger (8), the lumens of the needles (2,11) mutually communicate, through a passage (9) formed in the plunger (8) itself and opening onto the wider section (7a). Communication is likewise prevented by shifting the plunger (8) away from the first stroke end position, causing the passage (9) to be intercepted by the body (16).

## Description

The present invention relates to the field of the medical instruments and accessories, and specifically to a sampling device useful in vacutainer blood withdrawal systems.

At present, the system utilizing vacutainers, i.e. test tubes provided with a rubber cap and in which vacuum is provided beforehand for the withdrawal of blood samples to be forwarded to the biochemical analysis laboratories, is almost universally adopted. The withdrawal needle, to be inserted into the patient's vein, is mounted onto the convex side of a bell-shaped holder, a further needle for piercing the vacutainer rubber cap extending from the opposite side thereof. Thus, inserting a test tube inside the bell, a suction drawing the blood from the vase into the test tube itself is generated.

Since it is often necessary to collect a blood amount exceeding the capacity of an individual vacutainer, the needle piercing the vacutainer cap is provided with a small rubber seal that stops the blood outflow when a full vacutainer is extracted from the bell and replaced with an empty one, the withdrawal needle being kept inserted in the vein.

More specifically, said seal is shaped as a closed cap enveloping the needle, axially extending further thereto of about 2 mm. When the first test tube is inserted in the bell, the needle itself pierces the seal before entering the vacutainer cap. Therefore, the needle insertion causes the compression and the straining of the seal by the vacutainer cap, whereas when the vacutainer is extracted the seal is released, stretching over the needle tip and elastically sealing the lumen thereof.

A major drawback of this system is that, when replacing the vacutainer, due to the unavoidable inertia when the seal elastically recovers, the closing of the needle is not concomitant to the related extraction from the vacutainer. Therefore, within a certain time interval, however short, the needle lumen opens on the outside and a small blood amount can leak, depositing both on the outside of the seal and between the same and the needle. Unavoidably, as an effect of the seal "squashing" action exerted by the vacutainer, said blood amount, particularly the portion filling the clearance between the needle and the seal, deposits onto the outer surface of the cap of the subsequent vacutainer.

This can easily be verified by carefully checking the blood-filled vacutainers, onto the majority of which a blood droplet can often be noticed. The entailed danger is that during the subsequent transport and lab analysis steps of the sample, the exsiccated blood can uncontrolledly detach from the cap, dispersing in the environment. Moreover, at present, in order to avoid any contacts by the operators and potentially infected biological material and to prevent the forming of a biological aerosol, many analyzers do not open the vacutainer, piercing its cap with a needle and directly collecting the content thereof. Usually, the analyzer needle passes through the very spot where the above mentioned exsiccated blood residue may lie, and the possibility of the latter being at least partially intaken is anything but remote.

The leaking of small blood amounts can be favoured by the fact that during the withdrawal of several vacutainers the seal can be compressed and released several times, even seven or eight. Under these circumstances, the needle seldom pierces the seal on the same spot for all the vacutainers. Thus, the seal is pierced on different spots, and the actual sealing capability thereof is significantly reduced. Temperature may be another factor possibly hindering a swift sealing, since under cold temperatures the rubber becomes stiffer, hence slower in recovering the unstrained condition.

Another worrisome occurrence has to be highlighted, related as well to the fact that during the replacement of the vacutainer the patient's venous system is, within a certain time interval, freely and directly communicating with the outside. An examination of the blood residues onto the caps of the vacutainers, from the smaller 3 ml ones to the 6 and 10 ml ones, reveals different residue amounts thereon. Unexpectedly, since the sealing mechanism of the rubber seal is identical for all vacutainers and this should involve residues of substantially random amounts onto different vacutainers, the amount of blood deposited onto the cap of the smaller vacutainers is on average greater than that of the bigger ones.

This finding suggests that the venous system, at a certain stage of the withdrawal before being closed by the seal, and in particular when the vacutainer is extracted half-empty or in case of a troublesome operation, exerts a certain backsuction, directly proportional to the suction exerted by the vacutainer. This occurrence should be prevented at all costs, as it entails the taking into the venous system of already collected blood, or even of extraneous particles.

The main object of the present invention is to provide a blood withdrawal device useful in a blood withdrawal system utilizing vacutainers of the known type allowing to overcome the above described drawbacks, avoiding at all stages of the blood withdrawal a free and direct communication between the patient's venous system and the outside.

A specific object of the present invention is to provide a device of the aforementioned type, absolutely preventing small amounts of blood from depositing onto the outside of the vacutainers, or that a possible backsuction of the venous system can be in direct communication with the outside.

A further specific object of the present invention is to provide a device of the above mentioned type which has simple and reliable structure and operation, and therefore can be manufactured in a cost-effective manner.

Those above objects are achieved with the blood withdrawal device by means of a vacutainer system according to the present invention, the essential features of which are defined in the first of the annexed claims.

The features and the advantages of the blood withdrawal device by means of a vacutainer system according to the present invention will be made apparent by way of the following description of an embodiment thereof, given by way of example and not for limitative purposes, with reference to the annexed drawings wherein:
- Figure 1 is a schematic axial section view of the device according to the invention, in a closure configuration thereof;
- Figure 2 is a schematic axial section view of the device, in an open, or withdrawal, configuration thereof.

With reference to said figures, the device according to the present invention comprises a first cylinder 1, open at one end and closed at the opposite one by way of a bottom 1a. An eccentrically positioned withdrawal needle 2 axially extends from bottom 1a. The bottom end of needle 2 is sealingly engaged, e.g. thanks to a seal 3, within a hole 4 formed in bottom la, whereby the inside of cylinder 1 communicates with the outside through the lumen of needle 2 itself.

A second open cylinder 5, of a lesser diameter, is coaxially engaged within first cylinder 1, cooperating therewith so as to form a box-shaped body 16 defining a sealed chamber 7. More specifically, second cylinder 5 has a bottom 5a, extending from the open side of first cylinder 1 and with an externally convex shape. Onto the opposite side, the rim of second cylinder 5 is spaced from bottom la of first cylinder 1, so that chamber 7 comprises a first section 7a, of a greater diameter, adjacent to bottom la itself, and of a second section 7b, of a lesser diameter, adjacent to bottom 5a. The above mentioned rim is beveled, in order to form a frustoconical surface 6 connecting sections 7a and 7b of chamber 7 therebetween.

A box shaped plunger 8 is slidably and sealingly engaged within second cylinder 5. Plunger 8 axially slides within chamber 7 between a first and a second stroke end position determined by the abutment onto bottoms 1a, 5a, respectively. In fact, both the axial ends of the plunger 8 are closed, by a flat bottom 8a and a convex bottom 8b, fit to abut onto bottoms 1a, 5a of the two cylinders 1,5, respectively.

A passage 9 is radially formed in plunger 8, suitably positioned in order to communicate the inside of the latter with chamber 7. More specifically, passage 9 is formed near bottom 8a, so that, when plunger 8 is in the first stroke end position passage 9 is not intercepted by second cylinder 5 and faces wider section 7a of chamber 7. On the contrary, when plunger 8 slides towards the second stroke end position, passage 9 is intercepted by second cylinder 5, thereby sealing the inside of plunger 8.

Preferably, as it will be made apparent hereinafter, passage 9 is formed with a oblique path, in order to have an outline that, in the first stroke end position, locates itself with a generatrix just in correspondence to frusto-conical surface 6 of second cylinder 5, forming an extension of said surface towards the inside of plunger 8. Moreover, in order to keep passage 9 adjacent to the lumen outlet of drawing needle 2, the mutual rotation between plunger 8 and second cylinder 5 is prevented, e.g. by means of an axial guide, not shown, cooperating between the outer surface of plunger 8 and the inner surface of second cylinder 5.

Convex bottom 8b of plunger 8 has a central hole 10, within which the base end of a second needle 11 is sealingly engaged. The latter axially extends through a center hole 12 of bottom 5a of second cylinder 5, therefore the inside of plunger 8 opens on the outside through the lumen of needle 11. Further, a tubular seal 13 is preferably provided within hole 12 of bottom 5a, coaxially sheathing the second needle 11 and being integrally connected thereto in correspondence to the outer end, e.g. by a collar, not shown.

In use, body 16 of the sampling device according to the present invention is coupled to a bell-shaped holder 15 of known type, engaging first cylinder 1 within a hole 15a on the bottom of bell 15, so that the latter envelopes second cylinder 5 and needle 11. The coupling can be removable, e.g. with a screw or shrinking system, or integral. The latter system may be preferable because it prevents the reuse of the bell-shaped holder and, thus, avoids any possible contamination of needle 11.

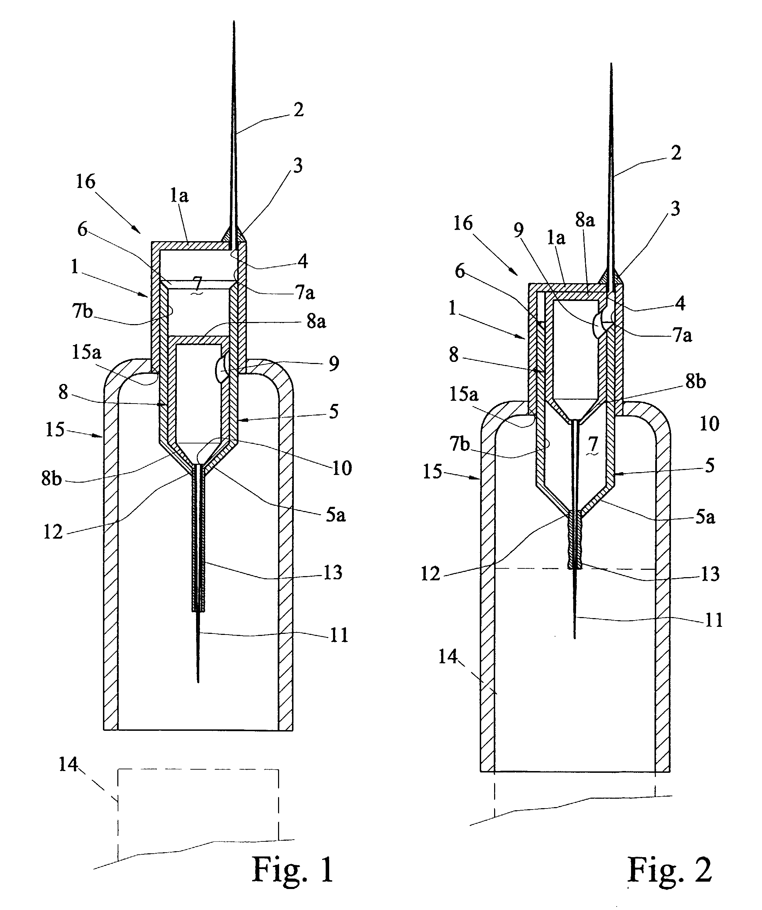

In order to carry out the sampling, the device is extracted from a sterilized packaging, inside which it is stored in the closure configuration shown in figure 1, corresponding to the second stroke end position of plunger 8, i.e. that abutting against bottom 5a of second cylinder 5. A vacutainer 14 of the conventional type, the outline thereof being depicted with a dashed line in the figures, is inserted from the side of the rubber cap, inside bell 15. Then the operator, while gripping bell 15 applies a slight axial pressure onto vacutainer 14, forcing it against needle 11.

At first this pressure induces the insertion of needle 11 into rubber cap of vacutainer 14, assisted by the resistance exerted by seal 13 onto the needle itself, and then, without piercing the cap, the sliding of plunger 8 towards the first stroke end position. When this position is reached, i.e. when the flat bottom 8a of plunger 8 abuts onto bottom 1a of first cylinder 1, withdrawal needle 2 is inserted in vein, and an effective pressure is applied onto vacutainer 14, until second needle 11 pierces the cap thereof. In this configuration, shown in Figure 2, the blood, drawn by the suction exerted by vacutainer 14, flows into plunger 8 through passage 9, and therefrom into the vacutainer itself through second needle 11. Tubular seal 13, strained between bottom 5a of second cylinder 5 and the cap of vacutainer 14, exerts a sealing action against possible fortuitous blood leaks.

Once filled, vacutainer 14 is drawn outwards, always holding bell 15. This, due to the friction between the rubber of the cap of vacutainer 14 and second needle 11, as well as to the elastic return thrust exerted by seal 13 onto needle 11 itself, draws back plunger 8 towards the second stroke end position, thus stopping the blood flow before that needle 11 exits the vacutainer. A further drawing of vacutainer 14 leads to its detachment from plunger 8 when the latter abuts onto convex bottom 5a of second cylinder 5.

It is apparent that along the entire procedure the venous system never directly opens on the outside. In fact, when needle 11 exits the vacutainer, the blood contained inside plunger 8, in this step sealingly closed, cannot possibly leak, nor backsuction phenomena can occur. This also applies for the filling of subsequent vacutainers 14, that is substantially carried out according to the same operative steps above disclosed. Thus the drawbacks of the withdrawal procedure according to the prior art are overcome.

The close proximity between passage 9 and the lumen outlet of withdrawal needle 2 prevents the occurrence of swirls possibly harmful to the globular elements of the blood. Frusto-conical surface 6 and the oblique outline of passage 9 further assist in preventing such occurrence, deviating the flow without discontinuities.

The entire device can be made of a transparent plastics material, thereby improving the operator's control of the withdrawal steps.

The presence of an elastic force hindering the movement of plunger 8 from the second to the first stroke end position is not strictly required for the device operation. However it is advantageous, in order to assist both the return of plunger 8 into the closure condition when vacutainer 14 is extracted, and the initial partial penetration of needle 11 into the rubber cap of the vacutainer tube itself.

In this respect, seal 13 advantageously couples the elastic hindering and the sealing functions, but it can be replaced by other equivalent means, e.g. a spring located inside chamber 7.

Due to its economical structure, the device according to the present invention can be cost-effectively manufactured, without prejudicing the reliability and the safety requirements thereof, that therefore can fully match those of the system according to the known art.

Variants and/or modifications can be brought to the blood withdrawal device by means of vacutainers according to the present invention, without departing from the protective scope thereof, as defined by the appended claims.

## Claims

1. A device for withdrawing blood by means of vacutainers (14), engaged or engageable with a holder having substantially the shape of a bell (15), said device comprising a withdrawal needle (2) axially extending externally to said bell (15) and a second needle (11) axially extending within said bell (15), for piercing a vacutainer (14), characterized in that it comprises: a box-shaped body (16), substantially coaxial to said bell (15), supporting at one end said withdrawal needle (2); a sealed chamber (7) defined within said body (16), comprising a wider end section (7a), communicating with the lumen of said withdrawal needle (2) and having a axial hole (12) at the opposite end thereof; a box-shaped plunger (8) sealingly sliding within said chamber (7), supporting said second needle (11) and communicating with the lumen thereof, said second needle (11) extending externally to said body (16) through said axial hole (12); and a passage (9) radially formed in said plunger (8), opening within said wider section (7a), for communicating the lumens of said needles (2, 11) therebetween, at a first stroke end position of said plunger (8), and intercepted by said body (16), in order to prevent said communication, as a consequence of the shifting of said plunger (8) away from said first stroke end position.

2. The device according to claim 1, comprising elastic means (13) for hindering the movement of said plunger (8) towards said fist stroke end position.

3. The device according to claims 1 or 2, comprising guide means cooperating between said body (16) and said plunger (8), for preventing the mutual rotation therebetween.

4. The device according to claim 3, wherein said passage (9) opens at a position radially corresponding to that of said withdrawal needle (2).

5. The device according to any one of the previous claims, wherein said body (16) is made of a first cylinder (1), of greater diameter, engaged or engageable to said bell (15) and supporting said withdrawal needle (2) and of a second cylinder (5), of lesser diameter, engaging within said first cylinder (1), so that said wider section (7a) of said chamber (7) is defined between a bottom (1a) of said first cylinder (1) and an end rim (6) of said second cylinder (5).

6. The device according to claim 5, wherein said rim (6) of said second cylinder is beveled, in order to form a frusto-conical surface (6) for jointing said wider section (7a) of said chamber (7) and the remaining section (7b) therebetween.

7. The device according to claim 6, wherein said passage (9) is formed with a oblique path, in order to have an outline which, in said first stroke end position, locates with a generatrix exactly in correspondence to said frusto-conical surface (6), forming an inward extension thereof within said plunger (8).

8. The device according to any one of claims 2 to 7, wherein said elastic hindering means (13) comprise a tubular seal (13) engaged within said axial hole (12) of said body (16), enveloping said second needle (11) along an axial portion thereof, and connected thereto at the outer end thereof.

9. The device according to any one of the previous claims, wherein said axial hole (12) is centrally formed in a convex bottom (5a) of said body (16).

10. The device according to any one of the previous claims, entirely made of a transparent plastics material.
